# EUROPÄISCHE PATENTSCHRIFT

(11) **EP 1 181 892 B1**
(45) Veröffentlichungstag und Bekanntmachung des Hinweises auf die Patenterteilung: **16.11.2005**
(21) Anmeldenummer: 01118056.9
(22) Anmeldetag: 25.07.2001
(51) Int. Cl.: A61B 5/12

(54) **Gerät zur elektromechanischen Stimulation und Prüfung des Gehörs**
Apparatus for electromechanical stimulation and auditory testing
Appareil de stimulation électromécanique et de test auditif

(30) Priorität: 25.08.2000 DE 10041725
(43) Veröffentlichungstag der Anmeldung: 27.02.2002
(73) Patentinhaber: PHONAK AG, 8712 Stäfa (CH)
(72) Erfinder: Leysieffer, Hans, Dr.-Ing., 82024 Taufkirchen (DE)
(74) Vertreter: Schwan - Schwan - Schorer

## Beschreibung

Die vorliegende Erfindung betrifft ein Gerät zur elektromechanischen Stimulation und Prüfung des Gehörs, mit einem elektromechanischen Wandler zum Erzeugen von mechanischen Schwingungen, die von außen durch den äußeren Gehörgang auf mindestens näherungsweise das Zentrum des Trommelfells (Umbo) und damit auf den Endpunkt des Hammergriffs nichtinvasiv übertragen werden..

Generell wird das Hörvermögen des Menschen derart überprüft, dass ein Schallsignal und damit eine akustische Welle über geeignete elektroakustische Einrichtungen dem Probanden monaural (einohrig) oder binaural (beidohrig) dargeboten werden und der Proband auf entsprechende Fragestellungen subjektiv reagiert, die dem jeweiligen Zweck der psychoakustischen Untersuchung adäquat sind. Diese elektroakustischen Einrichtungen werden mit dem Sammelbegriff Audiometer bezeichnet, wobei in den häufigsten Anwendungsfällen das Prüfsignal entweder auf elektronischem Weg erzeugt wird (analoge beziehungsweise digitale Signalgeneratoren) oder von einem geeigneten Tonträger (Magnetband, Compact Disc etc.) entnommen wird. Diese Prüfsignale werden dem Probanden auf akustischem Weg, zumeist über Lautsprecher unter sogenannten Freifeldbedingungen oder über speziell kalibrierte Mess-Kopfhörer dargeboten. In Sonderfällen werden diese akustischen Signale über kurze Schallleitungsschläuche und Ohrpassstücke zum äußeren Gehörgang geleitet, wenn zum Beispiel ein akustisch dicht abgeschlossenes Volumen vor dem Trommelfell für die spezielle Prüfung gefordert wird.

Darüber hinaus sind objektive Hörprüfungsverfahren (zum Beispiel BERA: "Brainstem Evoked Response Audiometry") bekannt, bei denen akustisch evozierte neuronale Antworten über Hautelektroden abgenommen und entsprechend analysiert werden. Ferner wird routinemäßig die Mobilität des Mittelohres so bestimmt, dass durch einen Druckaufbau im äußeren Gehörgang das Mittelohr ausgelenkt wird; parallel dazu werden über Lautsprecher über Zuleitungsschläuche ein oder mehrere Prüftöne zugeführt, und über entsprechende Wandler (Mikrofone und Druckmesser) werden Druck- und Schnelle im äußeren Gehörgang gemessen. Mit diesen Größen ist die akustische Impedanz und deren Veränderung bei pathologischen Veränderungen des Mittelohres bestimmbar. Durch entsprechende zeitliche Hüllkurvenkonfiguration und Pegelwahl ist auch die Auslösbarkeit des Stapediusreflexes messbar.

Bei allen diesen Verfahren wird grundsätzlich ein akustisches Signal dargeboten, welches auf bekannte Weise das Trommelfell in mechanische Schwingungen versetzt, die über die Gehörknöchelchenkette des Mittelohres zum Innenohr fortgeleitet und dort in ein neuronales Reizmuster umgewandelt werden, welches zu einem Höreindruck führt

Es sind ferner laseraudiometrische Untersuchungseinrichtungen, bei denen mit einem Laserdopplervibrometer optisch und damit berührungslos die dynamischen Bewegungen des Trommelfells beziehungsweise des Endpunktes des Hammergriffs, der sogenannte Umbo, auch bei sehr kleinen Bewegungsamplituden nahe der Ruhehörschwelle gemessen werden können (US-A-6 007 494).

Weiterhin existieren Ansätze, Untersuchungen des Mittelohres durch direkten Kontakt mit einem elektromechanischen Wandler vorzunehmen (DE-A-31 21 429 und A. Thullen: "Klinische Erfahrungen mit der Schallsonde nach Zöllner", Medizinal-Markt, Nr. 12, 1956, S. 444 bis 445). Dabei wird eine Schallsonde insbesondere invasiv bei Mittelohroperationen in Berührung mit dem Mittelohr gebracht. Ein zur präoperativen Demonstration für implantierbare Hörsysteme sowie zur psychoakustischen Messung der Ruhehörschwelle durch direkte mechanische Anregung des Umbo bestimmtes Gerät mit einem elektromechanischen Wandler zum Erzeugen von mechanischen Schwingungen im Audiobereich und einem starren mechanischen Koppelelement zum Übertragen der mechanischen Schwingungen ohne operativen Eingriff durch den äußeren Gehörgang in direktem mechanischem Kontakt auf das Zentrum des Trommelfells und damit auf den Hammergriff der Gehörknöchelchenkette des Mittelohres ist aus US-A-5 833 626 bekannt. In US-A-5 776 144 sind Systeme zur nichtinvasiven Ankopplung eines solchen Gerätes an die extrakorporale Trommelfellseite beschrieben.

Aus DE-C-198 21 602 ist ein Vibrationsmesskopf mit einem elektromechanischen Wandler bekannt, mittels dessen im ausschließlichen Resonanzbetrieb die Beweglichkeit der auf der aktorischen Wandlerseite angekoppelten schwingfähigen Elemente der Mittelohrstruktur durch eine zweite Messwicklung auswertbar ist, da sich die Dämpfung des Systems durch die Ankopplung an die Mittelohrstruktur als eine Veränderung der durch diese Spule erzeugten Spannung darstellt.

In dem in "Acoustica, Vol. 50 (1982) erschienenen Artikel "On Basic Research Towards an Improved Artificial Head for the Measurement of Hearing Protectors" von J. Schröter und H. Els wird über die Messung der mechanischen Impedanz von menschlichen Gewebeschichten und deren Nachbildung durch Kunststoff berichtet. Insbesondere befasst sich dieser Artikel mit einer Kunstkopfmethode zur Messung der Schalldämmung von Kapsel- und Stöpsel-Gehörschützem.

In dem in "The Journal of The Acoustical Society of America", Vol. 41, No. 5, 1967, Seiten 1220-1231 erschienenen Artikel "Dynamic Response of Middle-Ear Structures" von H. Fischler et al. wird von Impedanzmessungen an humanmedizinischen Präparationen berichtet.

In dem Artikel "Measurement of the eardrum impedance of human ears" von H. Hudde, J. Acoust. Soc. Am., 73 (1), Januar 1983, Seiten 242-247 wird über die Schwierigkeit der Messung der akustischen Impedanz im menschlichen Gehörkanal berichtet.

B. Feldman et al. berichten in "Measurement of Stapedial-Footplate Displacements during Transmission of Sound through the Middle Ear", The Journal of the Acoustical Society of America, Vol. 40, No. 6, 1966 über Messungen der Frequenzantwort der Vibration der stapedialen Fußplatte an Präparationen von menschlichem Leichenmaterial.

Über Messungen der Schalleitung in anatomischen Präparationen des menschlichen Temporalknochens wird ferner in "Measurement of Sound Transmission in the Middle Ear" von H. Fischler et al., Med. Electron. Biol. Engng., Vol. 2, Seiten 298-298, 1964 berichtet.

Insbesondere bei den beschriebenen objektiven Hörprüfungsverfahren (zum Beispiel BERA) bestehen in der akustischen Anregungsart einige Nachteile, wie zum Beispiel die durch die zumeist verwendeten elektrodynamischen oder elektromagnetischen Kopfhörer erzeugten magnetischen Felder. Diese magnetischen (Stör-)Felder führen zu Problemen bei der Vorverarbeitung und Analyse der von der Hautoberfläche des Kopfes elektrisch abgeleiteten evozierten Potentiale, die im nV-Bereich liegen können. Bei überschwellig monaural dargebotenen akustischen Signalen entsteht weiterhin bei mittleren bis höheren Schallpegeln das Problem des "Überhörens" des nicht untersuchten, kontralateralen Ohres durch akustische Schallabstrahlung des Kopfhörers beziehungsweise durch Körperschall (Knochenleitung), was zur Notwendigkeit der akustischen Vertäubung (Maskierung) dieses kontralateralen Ohres führt. Dieser Effekt ist bei zahlreichen psychoakustischen Fragestellungen unerwünscht, jedoch unvermeidlich. Nachteilig ist bei dem aus DE-C-198 21 602 bekannten Gerät insbesondere, dass eine breitbandige Messung nicht möglich ist, da das dort angewendete Verfahren auf Resonanzeffekten des Wandlers beruht. Eine echte Bestimmung der mechanischen Impedanz der angekoppelten Mittelohrstruktur ist so nicht möglich, insbesondere wenn Aussagen in verschiedenen spektralen Bereichen gewünscht sind. Messungen in verschiedenen spektralen Bereichen sind aber zumindest im Hauptsprachfrequenzbereich von erheblicher Bedeutung, wenn präzise Aussagen über den pathologischen Zustand des Mittelohres oder sogar des mechanisch daran gekoppelten Innenohres gemacht werden sollen.

Der Erfindung liegt die Aufgabe zugrunde, ein Gerät zur elektromechanischen Stimulation und Prüfung des Gehörs zu schaffen, das auf besonders zuverlässige Weise eine nichtinvasive objektive Überprüfung des Hörvermögens ermöglicht.

Diese Aufgabe wird dadurch gelöst, dass bei einem Gerät zur elektromechanischen Stimulation und Prüfung des Gehörs, mit einem elektromechanischen Wandler zum Erzeugen von mechanischen Schwingungen, die von außen durch den äußeren Gehörgang auf mindestens näherungsweise das Zentrum des Trommelfells (Umbo) und damit auf den Endpunkt des Hammergriffs nichtinvasiv übertragen werden, und einem Impedanzmesssystem zum Ermitteln der mechanischen Impedanz der an den Wandler angekoppelten biologischen Laststrukturdas Impedanzmesssystem eine Anordnung zum Messen der elektrischen Eingangsimpedanz des an die biologischen Laststruktur angekoppelten elektromechanischen Wandlers aufweist. Die Betrags- und Phasendaten dieser elektrischen Eingangsimpedanz spiegeln nämlich die angekoppelten Lastkomponenten wider, weil diese über die elektromechanische Kopplung des Wandlers transformiert auf der elektrischen Seite erscheinen und daher messbar sind.

Der vorliegend vorgesehene elektromechanische Wandler kann grundsätzlich für ein beliebiges bekanntes elektromechanisches Wandlungsprinzip ausgelegt sein. So kann es sich bei diesem Wandler insbesondere um einen elektromagnetischen, elektrodynamischen, magnetostriktiven, dielektrischen und insbesondere einen piezoelektrischen Wandler handeln.

Vorzugsweise ist dem elektromechanischen Wandler eine Treibereinheit vorgeschaltet, an welche der Wandler über einen Messwiderstand angeschlossen ist, und es ist ein Messverstärker vorgesehen, an dem als Eingangssignale die an dem Messwiderstand abfallende, dem Wandlerstrom proportionale Messspannung und die Wandlerklemmenspannung anliegen. Um Messverfälschungen vorzubeugen, wird zweckmäßig der Spannungsabfall an dem Messwiderstand hochohmig und massefrei abgegriffen, und der Messwiderstand ist vorteilhaft so bemessen ist, dass die Summe des Widerstandswertes des Messwiderstandes und des Betrages der komplexen elektrischen Eingangsimpedanz des an die biologischen Laststruktur angekoppelten elektromechanischen Wandlers groß gegenüber dem Innenwiderstand der Treibereinheit ist. Es sind ferner - vorzugsweise digitale - Mittel zur Bildung des Quotienten aus Wandlerklemmenspannung und Wandlerstrom vorgesehen.

Die beschriebene Impedanzmessung ist in keiner Weise auf eine einzige Messfrequenz oder einen einzigen Messpegel beschränkt. Vorteilhaft sind - vorzugsweise digitale - Mittel zum Ermitteln der mechanischen Impedanz der im implantierten Zustand an den Wandler angekoppelten biologischen Laststruktur in Abhängigkeit von der Frequenz und/oder dem Pegel des von dem Wandler abgegebenen Stimulationssignals vorgesehen. Gerade durch Messungen über den gesamten Übertragungsfrequenzbereich und Stimulationspegelbereich können wichtige Detailaussagen über lineare und insbesondere nichtlineare Variationen des Mittel und/oder Innenohres gewonnen werden. So kann beispielsweise erwartet werden, dass eine mechanische Nichtlinearität des Mittelohres zum Beispiel aufgrund einer teilweisen Luxation eines Mittelohrossikels durch elektrische Pegelvariation der Impedanzmessung ermittelt werden kann. Wenn auf ein Pegelanalyse bezüglich Nichtlinearitäten über den ganzen Pegelnutzbereich verzichtet wird und das Messsystem empfindlich und rauscharm genug ausgelegt ist, kann die elektrische Wandlerimpedanzmessung auch unterhalb der Ruhehörschwelle des individuellen Patienten erfolgen, um ihn nicht durch die Messsignale zu stören.

In weiterer Ausgestaltung der Erfindung können - vorzugsweise digitale - Mittel zum Ermitteln der spektralen Lage von Resonanzfrequenzen in dem Verlauf der gemessenen

Impedanz über der Stimulationsfrequenz sowie zum Ermitteln der Differenz zwischen den bei den Resonanzfrequenzen auftretenden Impedanzmesswerten vorgesehen sein. Diese Differenz gibt Auskunft über die mechanischen Schwinggüten.

Vorzugsweise ist ein passives mechanisches Koppelelement zum Ankoppeln des elektromechanischen Wandlers an den Umbo vorgesehen.

In weiterer Ausgestaltung der Erfindung ist elektromechanische Wandler in einem Gehäuse untergebracht ist, dessen geometrische Abmessungen so dimensioniert sind, dass bei Platzierung des Wandlers in dem Eingangsbereich des äußeren Gehörgangs die untersuchende Person auch unter Verwendung eines Mikroskopes freie Sicht auf das den Umbo mechanisch kontaktierende Wirkende des Koppelelementes behält.

Die Koppelstange ist vorzugsweise manuell leicht biegbar gestaltet, so dass sie den individuellen geometrischen Formen des äußeren Gehörgangs problemlos angepasst werden kann. Zweckmäßig ist ferner das Koppelelement mit dem elektromechanischen Wandler nicht mechanisch fest, sondern lösbar, insbesondere über eine mechanische Steckverbindung, verbunden. Dies erlaubt zum Beispiel den Einsatz unterschiedlicher Koppelelemente, die aus hygienischen Gründen leicht austauschbar und als Einwegartikel ausführbar sind.

Der elektromechanische Wandler ist in Verbindung mit dem mechanischen Koppelelement vorteilhaft so ausgeführt, dass die erste mechanische Resonanzfrequenz am oberen Ende des spektralen Übertragungsbereiches von ≥ 10 kHz liegt. Aufgrund einer solchen Breitbandigkeit werden kurze Einschwingzeiten erreicht.

In weiterer Ausgestaltung der Erfindung ist der elektromechanische Wandler so ausgeführt, dass seine mechanische Quellimpedanz im gesamten spektralen Übertragungsbereich deutlich größer ist als die mechanische Lastimpedanz, die durch das aus Trommelfell, Gehörknöchelchenkette und Innenohr bestehende biologische System gebildet wird. Auf diese Weise wird eine von den individuellen Schwankungen dieser biologischen Lastimpedanz unabhängige Einprägung der Auslenkung des Wirkendes des Koppelelementes erreicht.

Vorzugsweise sind ein den Wandler treibender Treiber und der Wandler selbst so ausgelegt, dass der Wandler mit Koppelelement im gesamten spektralen, audiologischen Übertragungsbereich bei mechanisch angekoppelter Gehörknöchelchenkette maximale Auslenkungsamplituden im Bereich von 1 bis 5 µm erzeugt, die einem äquivalenten Schalldruckpegel von 120 bis 140 dB SPL entsprechen.

Der elektromechanische Wandler ist vorteilhaft im Hinblick auf eine Minimierung des von schwingenden Wandlerstrukturen abgestrahlten Schallsignals gekapselt. Dadurch kann gegebenenfalls selbst bei hohen Stimulationspegeln auf eine akustische Vertäubung des kontralateralen, nicht untersuchten Ohres verzichtet werden.

Das Gerät kann ferner für eine simultane, beidohrige Stimulation und Gehörprüfung doppelt ausgeführt sein.

Bevorzugte Ausführungsbeispiele des erfindungsgemäßen Gerätes zur elektromechanischen Stimulation und Prüfung des Gehörs sind nachstehend unter Bezugnahme auf die beiliegenden Zeichnungen näher beschrieben. Es zeigen:
- FIG. 1: ein Ausführungsbeispiel einer Anordnung zur objektiven Prüfung des Gehörs,
- FIG. 2: beispielhaft eine mögliche Ausführungsform des Impedanzmesssystems der Anordnung gemäß FIG. 1,
- FIG. 3: ein elektromechanisches Ersatzschaltbild für die Näherung eines piezoelektrischen Wandlers mit angekoppelten biologischen Lastkomponenten,
- FIG. 4: ein Ersatzschaltbild der elektrischen Wandlerimpedanz Z_{L} entsprechend FIG. 3,
- FIG. 5: den Verlauf des Betrages der elektrischen Wandlerimpedanz /Z_{L}/ über der Frequenz f gemäß FIG. 4 in doppeltlogarithmischer Darstellung,

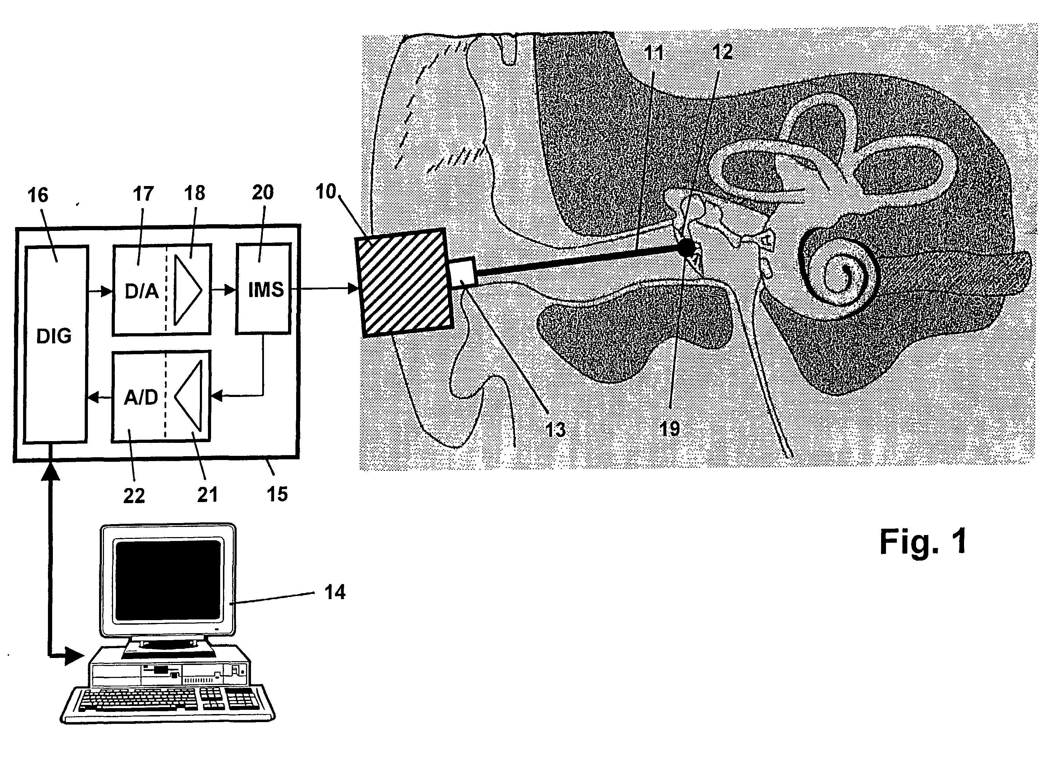

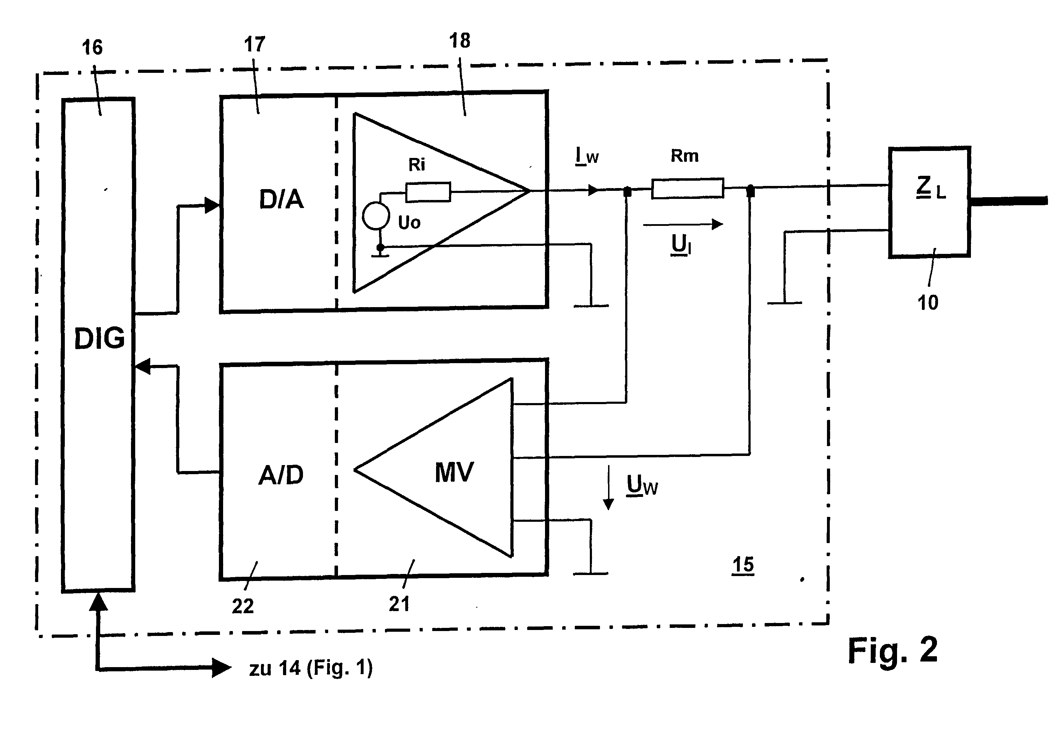

Die Anordnung gemäß FIG. 1 weist einen elektromechanischen Wandler 10 zum Erzeugen von mechanischen Schwingungen und ein Koppelelement 11 in Form einer Koppelstange auf, über welche diese Schwingungen auf den Endpunkt des Hammergriffs im Zentrum des Trommelfells (Umbo) 12 übertragen werden. Die Koppelstange 11 kann mit der aktorischen Ausgangsseite des Wandlers 10 fest oder, zum Beispiel über eine bei 13 angedeutete Steckverbindung, lösbar verbunden sein. Das von dem Wandler 10 abgewandte Wirkende 19 der Koppelstange 11 ist konstruktiv so ausgeführt, dass ein verletzungsfreier mechanischer Kontakt zum Umbo gewährleistet ist. Zum Positionieren des Wandlers 10 und des Koppelelements 11 können zweckmäßig Anordnungen verwendet werden, wie sie aus der oben genannten US-A-5 776 144 bekannt sind.

Die den Wandler treibenden elektrischen Signale werden bevorzugt mit einem Rechner 14 digital erzeugt und einem Hardware-Interface 15 zum Beispiel über eine serielle Standardschnittstelle wie RS 232 übermittelt. Das Interface 15 enthält eine digitale Schnittstelle (DIG) 16, welche die digital erzeugten Signale des Rechners 14 an einen D/A-Wandler 17 mit einem nachfolgenden Treiber 18 überträgt, der dem jeweiligen Wandlerprinzip des elektromechanischen Wandlers 10 angepasst ist. Zwischen Treiber 18 und den elektromechanischen Wandler 10 ist ein Impedanzmesssystem (IMS) 20 zur analogen Ermittlung der elektrischen Wandlerimpedanz geschaltet. Die von dem Impedanzmesssystem 20 abgegebenen analogen Messdaten werden über einen Messverstärker 21 und einene zugehörigen A/D-Wandler 22 in digitale Messdaten umgeformt und über das digitale Schnittstelle 16 dem Rechner 14 zur weiteren Verarbeitung, Auswertung, Darstellung und Speicherung zur Verfügung gestellt.

FIG. 2 zeigt eine mögliche einfache Ausführungsform des Impedanzmesssystems 20 gemäß FIG. 1. Die von der digitalen Schnittstelle 16 kommenden digitalen Treiberdaten für den elektromechanischen Wandler 10 werden von dem D/A-Wandler 17 in ein analoges Signal verwandelt und dem Wandlertreiber 18 zugeführt. Im vorliegenden Beispiel ist der Ausgang des Treibers 18 als Spannungsquelle Uₒ mit dem Innenwiderstand Rᵢ dargestellt. Das analoge Ausgangssignal dieses Treibers 18 wird dem eine komplexe elektrische Impedanz Z_{L} aufweisenden elektromechanischen Wandler 10 über einen Messwiderstand Rₘ zugeführt.

Ist die Summe von Rₘ und dem Betrag von Z_{L} groß gegen Rᵢ, dann erfolgt eine Spannungseinprägung auf den elektromechanischen Wandler 10. Greift man den Spannungsabfall an Rₘ mit dem dargestellten Messverstärker (MV) 21 entsprechend hochohmig und massefrei ab, steht eine dem Wandlerstrom I_{W} proportionale Messspannung U_{I} zur Verfügung. Gleichzeitig steht dem Messverstärker 21 die Wandlerklemmenspannung U_{w} zur Verfügung. Durch entsprechende A/D-Wandlung dieser Messspannungen in dem A/D-Wandler 22 stehen der digitalen Schnittstelle 16 beide Datensätze digital zur Verfügung. Durch entsprechende digitale Quotientenbildung ist somit die Ermittlung der komplexen elektrischen Wandlerimpedanz Z_{L} = U_{W}/I_{W} nach Betrag und Phase möglich.

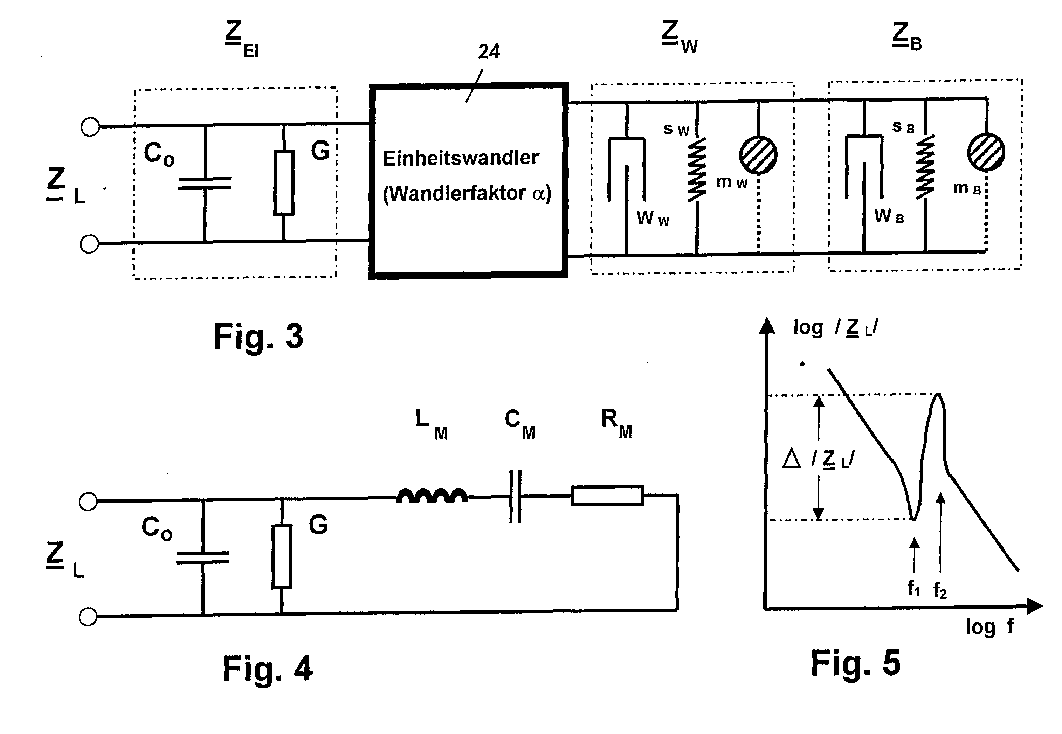

FIG. 3 zeigt in einem elektromechanischen Ersatzschaltbild die Näherung eines piezoelektrischen Wandlers mit angekoppelten biologischen Lastkomponenten. Der piezoelektrische Wandler wird auf der elektrischen Impedanzseite Z_{EI} im wesentlichen durch die Ruhekapazität Cₒ und einen Verlustleitwert G bestimmt. Auf einen elektromechanischen Einheitswandler 24 mit einem elektromechanischen Wandlerfaktor α folgen die mechanischen Komponenten des Wandlers selbst, die die mechanische Impedanz Z_{W} darstellen. Wird ein piezoelektrischer Wandler hochabgestimmt betrieben, das heißt, liegt die erste mechanische Resonanzfrequenz am oberen Ende des spektralen Übertragungsbereiches, wie dies in US-A-5 277 694 näher erläutert ist, dann wird die mechanische Impedanz des Wandlers Z_{W} in erster Näherung gut durch die mechanischen Komponenten dynamische Wandlermasse mw, Wandlersteifigkeit sw und den Wandlerreibwiderstand (realer Anteil) W_{W} bestimmt. Auch die biologische, mechanische Lastimpedanz Z_{B} soll im vorliegenden Beispiel durch die drei mechanischen Impedanzkomponenten Masse m_{B} (zum Beispiel Masse der Mittelohrossikel), Steifigkeit s_{B} (zum Beispiel Steifigkeit der die Mittelohrossikel haltenden Bänder und des einspannenden Ringbandes der Steigbügelfußplatte im ovalen Fenster) und Reibwiderstand W_{B} angenähert sein. Unter der Annahme, dass auf der mechanischen Lastseite sowohl die Wandler- wie auch die biologischen Lastkomponenten diesselbe Schnelle erfahren (mechanische Parallelschaltung), ergibt sich nach Transformation der mechanischen Komponenten durch den Einheitswandler 24 auf die elektrische Seite ein elektrisches Ersatzschaltbild, das in FIG. 4 dargestellt ist.

FIG. 4 zeigt das Ersatzschaltbild der elektrischen Wandlerimpedanz Z_{L} entsprechend FIG. 3, wobei die Spule L_{M} die Summe der Massen M_{W} und M_{B} widerspiegelt, die Kapazität C_{W} die mechanische Parallelschaltung der Steifigkeiten s_{W} und s_{B} und der Widerstand R_{M} die mechanische Parallelschaltung der Anteile W_{W} und W_{B}.

FIG. 5 zeigt den Verlauf des Betrages der elektrischen Wandlerimpedanz /Z_{L}/ über der Frequenz f gemäß FIG. 4 in doppeltlogarithmischer Darstellung. Man erkennt einen grundsätzlich kapazitiven Verlauf von /Z_{L}/, der durch Cₒ bestimmt wird. Die auftretende Serienresonanz bei f₁ und die Parallelresonanz bei f₂ werden durch die Komponenten L_{M} und C_{M} mit Cₒ bestimmt. Die Größe Δ /Z_{L}/ gibt Auskunft über die mechanische Schwinggüte. Somit können aus der spektralen Lage von f1 und f2 und der Größe Δ /Z_{L}/ sehr genaue Informationen über die biologischen Lastkomponenten gewonnen werden, insbesondere wenn die Impedanzmessungen den ganzen spektralen und Pegelbereich des Hörbereiches repräsentieren (zum Beispiel 20 Hz bis 15 kHz, äquivalenter Anregungspegel von der Ruhehörschwelle (ca. 0 bis 40 dB SPL) bis zur Unbehaglichkeitsgrenze entsprechend etwa 100 bis 120 dB SPL). In FIG. 5 sind Resonanzen höherer Ordnung nicht dargestellt, die jedoch sicherlich bei realer Messung auftreten, aber prinzipiell den gleichen transformierenden Gesetzmäßigkeiten genügen wie hier beschrieben, damit genauso präzise messbar sind und so verfeinerte Aussagen über die zu messende biologische Laststruktur möglich sind.

## Patentansprüche

1. Gerät zur elektromechanischen Stimulation und Prüfung des Gehörs, mit einem elektromechanischen Wandler (10, 30) zum Erzeugen von mechanischen Schwingungen, die von außen durch den äußeren Gehörgang auf mindestens näherungsweise das Zentrum des Trommelfells (Umbo) und damit auf den Endpunkt des Hammergriffs nichtinvasiv übertragen werdenund einem Impedanzmesssystem (20, 31) zum Ermitteln der mechanischen Impedanz der an den Wandler (10, 30) angekoppelten biologischen Laststruktur, **dadurch gekennzeichnet, dass** das Impedanzmesssystem (20) eine Anordnung zum Messen der elektrischen Eingangsimpedanz des an die biologischen Laststruktur angekoppelten elektromechanischen Wandlers (10) aufweist.

2. Gerät nach Anspruch 1, **dadurch gekennzeichnet, dass** dem elektromechanischen Wandler (10) ein Treiber (18) vorgeschaltet ist, der Wandler an den Treiber über einen Messwiderstand (Rₘ) angeschlossen ist und ein Messverstärker (21) vorgesehen ist, an dem als Eingangssignale die an dem Messwiderstand (Rₘ) abfallende, dem Wandlerstrom (I_{w}) proportionale Messspannung (U_{I}) und die Wandlerklemmenspannung (U_{W}) anliegen.

3. Gerät nach Anspruch 2, **dadurch gekennzeichnet, dass** der Spannungsabfall (U_{I}) an dem Messwiderstand (Rₘ) hochohmig und massefrei abgegriffen wird.

4. Gerät nach Anspruch 2 oder 3, **dadurch gekennzeichnet, dass** der Messwiderstand (Rₘ) so bemessen ist, dass die Summe des Widerstandswertes (Rₘ) des Messwiderstandes und des Betrages der komplexen elektrischen Eingangsimpedanz (Z_{L}) des an die biologischen Laststruktur angekoppelten elektromechanischen Wandlers (10) groß gegenüber dem Innenwiderstand (Rᵢ) des Treibers (18) ist.

5. Gerät nach einem der Ansprüche 2 bis 4 **gekennzeichnet durch** - vorzugsweise digitale - Mittel (14, 16) zur Bildung des Quotienten aus Wandlerklemmenspannung (U_{W}) und Wandlerstrom (I_{w}).

6. Gerät nach einem der vorhergehenden Ansprüche **gekennzeichnet durch** - vorzugsweise digitale - Mittel (14, 16) zum Ermitteln der mechanischen Impedanz der im implantierten Zustand an den Wandler (10, 30) angekoppelten biologischen Laststruktur in Abhängigkeit von der Frequenz und/oder dem Pegel des von dem Wandler abgegebenen Stimulationssignals, sowie vorzugsweise zum Ermitteln der spektralen Lage von Resonanzfrequenzen (f1 und f2) in dem Verlauf der gemessenen Impedanz über der Stimulationsfrequenz (f) und vorzugsweise zum Ermitteln der Differenz Δ/Z_{L}/ zwischen den bei den Resonanzfrequenzen (f1 und f2) auftretenden Impedanzmesswerten.

7. Gerät nach einem der vorhergehenden Ansprüche, **dadurch gekennzeichnet, dass** zum Ankoppeln des elektromechanischen Wandlers (10, 30) an den Umbo ein passives mechanisches Koppelelement (11) vorgesehen ist, das vorzugsweise als in axialer Richtung bezüglich der zu übertragenden Schwingungen steife Koppelstange ausgebildet ist, deren von dem Wandler (10, 30) abgewandtes Wirkende konstruktiv so ausgeführt ist, dass ein verletzungsfreier mechanischer Kontakt zum Umbo gewährleistet ist.

8. Gerät nach Anspruch 7, **dadurch gekennzeichnet, dass** der elektromechanische Wandler (10, 30) in einem Gehäuse (28) untergebracht ist, dessen geometrische Abmessungen so dimensioniert sind, dass bei Platzierung des Wandlers in dem Eingangsbereich des äußeren Gehörgangs die untersuchende Person auch unter Verwendung eines Mikroskopes freie Sicht auf das den Umbo mechanisch kontaktierende Wirkende des Koppelelementes behält.

9. Gerät nach einem der Ansprüche 7 bis 8, **dadurch gekennzeichnet, dass** das Koppelelement (11) mit dem elektromechanischen Wandler (10, 30) lösbar, insbesondere über eine mechanische Steckverbindung (13), verbunden ist.

10. Gerät nach einem der vorhergehenden Ansprüche, **dadurch gekennzeichnet, dass** der elektromechanische Wandler (10, 30) in Verbindung mit dem mechanischen Koppelelement (11) so ausgeführt ist, dass die erste mechanische Resonanzfrequenz am oberen Ende des spektralen Übertragungsbereiches von ≥ 10 kHz liegt, wobei die mechanische Quellimpedanz des Wandlers vorzugsweise im gesamten spektralen Übertragungsbereich deutlich größer ist als die mechanische Lastimpedanz, die durch das biologische System Trommelfell, Gehörknöchelchenkette und Innenohr gebildet wird.

11. Gerät nach einem der Ansprüche 7 bis 10, **gekennzeichnet**, dass ein den Wandler (10, 30) treibender Treiber (18) und der Wandler selbst so ausgelegt sind, dass der Wandler mit Koppelelement (11) im gesamten spektralen, audiologischen Übertragungsbereich bei mechanisch angekoppelter Gehörknöchelchenkette maximale Auslenkungsamplituden im Bereich von 1 bis5 µm erzeugt, die einem äquivalenten Schalldruckpegel von 120 bis 140 dB SPL entsprechen.

## Claims

1. Device for electromechanical stimulation and testing of hearing, comprising an electromechanical transducer (10, 30) for producing mechanical vibrations, which are non-invasively transmitted from the outside through the external auditory canal to at least approximately the centre of the tympanic membrane (umbo) and thus to the manubrium mallei; and an impedance measuring system (20, 31) for determining the mechanical impedance of the biological load structure which is coupled to the transducer (10, 30), **characterised in that** the impedance measuring system (20) comprises means for measuring the electrical input impedance of the electromechanical transducer (10) coupled to the biological load structure.

2. Device as claimed in claim 1, **characterised in that** a driver (18) is connected upstream the electromechanical transducer (10), that the transducer is connected to the driver via a measuring resistance (Rₘ), and that a measuring amplifier (21) is provided, which has applied thereto, as input signals, the measuring voltage (U_{I}) across the measuring resistance (Rₘ), which measuring voltage is proportional to the transducer current (I_{w}), and the transducer terminal voltage (U_{W}).

3. Device as claimed in claim 2, **characterised in that** the measuring voltage (U_{I}) across the measuring resistance (Rₘ) is taken off with floating ground and at high-impedance.

4. Device as claimed in claim 2 or 3, **characterised in that** the measuring resistance (Rₘ) is dimensioned such that the sum of the resistance value (Rₘ) of the measuring resistance and of the absolute value of the complex electrical input impedance (Z_{L}) of the electromechanical transducer (10) coupled to the biological load structure is large with respect to the internal resistance o(Rᵢ) of the driver unit (18).

5. Device as claimed in any one of claims 2 to 4, **characterised by** - preferably digital - means (14, 16) for providing the quotient of the transducer terminal voltage (U_{W}) and of the transducer current (I_{w}).

6. Device as claimed in any one of the preceding claims, **characterised by** - preferably digital - means (14, 16) for measuring the mechanical impedance of the biological load structure which in the implanted state is coupled to the transducer (10. 30) as a function of the frequency and/or the level of a stimulation signal delivered by the transducer, and preferably for determining the spectral distribution of resonance frequencies (f1 and f2) in the course of the measured impedance as a function of the frequency (f) of stimulation and preferably for determining the difference Δ/Z_{L}/ between the impedance measurement values occurring at the resonance frequencies (f1 and f2).

7. Device as claimed in any one of the preceding claims, **characterised in that** there is provided a passive mechanical coupling element (11) for coupling the electromechanical transducer (10, 30) to the umbo, which coupling element (11) preferably is designed as a coupling rod which is stiff in axial direction with respect to the vibrations to be transmitted thereby, and the end of which that is remote from the transducer (10, 30) is designed for a non-traumatic mechanical contact with the umbo.

8. Device as claimed in claim 7, **characterised in that** the electromechanical transducer (10, 30) is disposed within a housing (28), the geometrical dimensions of which are selected such that, upon placing the transducer in the entrance region of the external auditory canal, an unobstructed view onto the end of the coupling element which mechanically contacts the umbo is preserved, also when using a microscope.

9. Device as claimed in any one of claims 7 to 8, **characterised in that** the coupling element (11) is detachably connected, preferably by means of a mechanical plug connection (13), to the electromechanical transducer (10, 30).

10. Device as claimed in any one of the preceding claims, **characterised in that** the electromechanical transducer (10, 30) in combination with the mechanical coupling element (11) is designed such that the first mechanical resonance frequency is at the upper end of the spectral transmission range of ≥ 10 kHz, wherein the mechanical source impedance of the transducer, in the entire spectral transmission range, preferably is distinctly higher than the mechanical load impedance which is formed by the biological system of tympanic membrane, ossicular chain and inner ear.

11. Device as claimed in any one of claims 7 to 10, **characterised in that** a driver driving the electromechanical transducer (10, 30) and the transducer itself are designed such that the transducer together with the mechanical coupling element (11) and with the ossicular chain being mechanically coupled thereto, in the entire spectral audiologic transmission range, has maximum amplitudes of deflection of from 1 to 5 µm corresponding to an equivalent sound pressure level of from 120 to 140 dB SPL.

## Revendications

1. Appareil destiné à la stimulation électromécanique et à un test auditif, comportant un convertisseur (10, 30) électromécanique destiné à générer des vibrations mécaniques, qui sont transmises de manière non invasive de l'extérieur à travers le conduit auditif externe vers au moins approximativement le centre de la membrane du tympan et donc vers le point d'extrémité du manche du marteau, et un système de mesure de l'impédance (20, 31) destiné à déterminer l'impédance mécanique de la structure de charge biologique couplée au convertisseur (10, 30), **caractérisé en ce que** le système de mesure de l'impédance (20) comporte un dispositif de mesure de l'impédance électrique d'entrée du convertisseur électromécanique (10) couplé à la structure de charge biologique.

2. Appareil selon la revendication 1, **caractérisé en ce qu'**un excitateur (18) est monté en amont du convertisseur électromécanique (10), le convertisseur est couplé à l'excitateur (18) par l'intermédiaire d'une résistance de mesure (Rₘ) et il est prévu un amplificateur de mesure (21) sur lequel sont appliquées sous forme de signaux d'entrée la tension de mesure (U_{I}), proportionnelle au courant du convertisseur (I_{w}) et en chute sur la résistance de mesure (Rₘ), et la tension aux bornes du convertisseur (U_{w}).

3. Appareil selon la revendication 2, **caractérisé en ce que** la chute de tension (U_{I}) sur la résistance de mesure (Rₘ) est prélevée avec une haute impédance et sans masse.

4. Appareil selon la revendication 2 ou 3, **caractérisé en ce que** la résistance de mesure (Rₘ) est dimensionnée de telle sorte que la somme, formée par la valeur de résistance (Rₘ) de la résistance de mesure (Rₘ) et la valeur de l'impédance d'entrée (Z_{L}) électrique complexe du convertisseur électromécanique (10) couplé à la structure de charge biologique, est élevée par rapport à la résistance intérieure (Rᵢ) de l'excitateur (18).

5. Appareil selon l'une quelconque des revendications 2 à 4, **caractérisé par** des moyens (14, 16) - de préférence numériques - destinés à former le quotient à partir de la tension aux bornes du convertisseur (U_{w}) et du courant du convertisseur (I_{w}).

6. Appareil selon l'une quelconque des revendications précédentes, **caractérisé par** des moyens (14, 16) - de préférence numériques - destinés à déterminer l'impédance mécanique de la structure de charge biologique, en position implantée, couplée au convertisseur (10, 30) en fonction de la fréquence et/ou du niveau du signal de stimulation émis par le convertisseur, et de préférence destinés à déterminer la position spectrale de fréquences de résonance (f1 et f2) dans la courbe de l'impédance mesurée par l'intermédiaire de la fréquence de stimulation (f) et, de préférence, destinés à déterminer la différence Δ/Z_{L}/ entre les valeurs de mesure de l'impédance se formant aux fréquences de résonance (f1 et f2).

7. Appareil selon l'une quelconque des revendications précédentes, **caractérisé en ce qu'**il est prévu un élément de couplage (11) mécanique passif pour coupler le convertisseur (10, 30) électromécanique à la membrane du tympan, lequel élément est conçu sous forme de tige de couplage rigide dans le sens axial par rapport aux vibrations à transmettre, dont l'extrémité active, opposée au convertisseur (10, 30), est configurée de manière à garantir un contact mécanique sans blessure avec la membrane du tympan.

8. Appareil selon la revendication 7, **caractérisé en ce que** le convertisseur (10, 30) électromécanique est logé dans un boîtier (28) dont les dimensions géométriques sont choisies de telle sorte que, lors de la mise en place du convertisseur dans la zone d'entrée du conduit auditif externe, la personne effectuant l'examen peut aussi voir librement, moyennant l'utilisation d'un microscope, l'extrémité active de l'élément de couplage en contact mécanique avec la membrane du tympan.

9. Appareil selon l'une quelconque des revendications 7 à 8, **caractérisé en ce que** l'élément de couplage (11) est assemblé de manière amovible avec le convertisseur (10, 30) électromécanique, en particulier par l'intermédiaire d'un assemblage enfiché (13) mécanique.

10. Appareil selon l'une quelconque des revendications précédentes, **caractérisé en ce que** le convertisseur (10, 30) électromécanique assemblé à l'élément de couplage (11) mécanique est conçu de telle sorte que la première fréquence de résonance mécanique se situe à l'extrémité supérieure du domaine de transmission spectral de ≥ 10 kHz, l'impédance de source du convertisseur, de préférence dans l'ensemble du domaine de transmission spectral, étant nettement supérieure à l'impédance de charge mécanique, qui est formée par le système biologique constitué du tympan, de la chaîne tympano-ossiculaire et de l'oreille interne.

11. Appareil selon l'une quelconque des revendications 7 à 10, **caractérisé en ce qu'**un excitateur (18) excitant le convertisseur (10, 30) et le convertisseur lui-même sont réalisés de telle sorte que, lorsque la chaîne tympano-ossiculaire est couplée mécaniquement, le convertisseur (10, 30), conjointement avec l'élément de couplage (11), génère dans l'ensemble du domaine de transmission audiologique spectral des amplitudes de déviation maximales dans le domaine de 1 à 5 µm qui correspondent à un niveau de pression acoustique équivalent de 120 à 140 dB SPL.
